# EUROPEAN PATENT SPECIFICATION

(11) **EP 4 470 530 B1**
(45) Date of publication and mention of the grant of the patent: **02.09.2026**
(21) Application number: 23214455.0
(22) Date of filing: 05.12.2023
(51) Int. Cl.: A61K 9/48

(54) **AN ORAL SOFT CAPSULE THAT ENCAPSULATES A NALBUPHINE FORMULATION**
WEICHE ORALKAPSEL ZUR VERKAPSELUNG EINER NALBUPHINFORMULIERUNG
CAPSULE MOLLE ORALE ENCAPSULANT UNE FORMULATION DE NALBUPHINE

(30) Priority: 31.05.2023 US 202318203863
(43) Date of publication of application: 04.12.2024
(73) Proprietor: PhytoHealth Corporation, Taipei, Taiwan 105 (TW)
(72) Inventor: LEE, I-Lin, 105 Taipei City (TW); WANG, Teng-Hsu, 105 Taipei City (TW)
(74) Representative: Straus, Alexander

(56) References cited:
- US-A1- 2003 104 048
- US-A1- 2016 279 056

## Description

### TECHNOLOGY FIELD

The present invention relates to an oral soft capsule that encapsulates a nalbuphine formulation.

### BACKGROUND OF THE INVENTION

Nalbuphine is an analgesic that acts on opioid receptors. However, it has a different pharmacological mechanism from conventional opioid analgesics (such as morphine), and has both efficacious and antagonistic effects on opioid receptors, respectively, µ-receptor antagonist and κ-receptor agonist. Nalbuphine has no respiratory depression which is serious side effect induced by morphine, and extremely low addiction potential. Therefore, nalbuphine has widely been used clinically. It has been reported that nalbuphine is used to relieve moderate to severe pain and as a supplement to balanced anesthesia, such as anesthesia before and after surgery, and obstetric anesthesia during labor pains. In addition, it also has a good analgesic effect on pain relief for migraine, toothache and cancer patients (Beaver et al., Analgesic effect of intramuscular and oral nalbuphine in postoperative pain, Clinical Pharmacology and Therapeutics (1981) 29, 174-180; Errick and Heel, A Preliminary Review of its Pharmacological Properties and Therapeutic Efficacy, Drugs (1983) 26, 1991-211; Kay et al., Oral nalbuphine for the treatment of pain after dental extractions, Br. J. Anaesth. (1988), 61, 313-317; Hoskin and Hanks, Opioid agonist-antagonist drugs in acute and chronic pain states, Drugs (1991) 41(3): 326-44). However, in the past, nalbuphine could only be administered by intravenous, intramuscular or subcutaneous injection, but it could not be orally administered. However, oral administration is the most ideal way.

In order to solve the problem that nalbuphine cannot be administered orally, it is disclosed in Taiwan Patent No. I226239 that nalbuphine ester prodrug is used as an oral pharmaceutical composition to solve the problem of nalbuphine as not being applicable for oral administration. Further, an oral pharmaceutical composition using appropriate excipients to improve the low oral bioavailability of nalbuphine is disclosed in Taiwan Patent No. I397413, which provides a specific formula composition, for example, the composition set forth in claim 13, comprising 10 to 300 mg of nalbuphine, 12.5 to 50 mg of isorhamnetin, 120 mg to 10 g of polyethylene glycol 400 (PEG 400) and 120 mg to 2.5 g of Tween 20 as excipients. However, because the excipients as used are hydrophilic, there are some problems caused, for example, the capsule shell will become hard after being made into capsules, the disintegration will delay, and the disintegration of the active ingredient will be reduced. US 2003104048 A1 discloses a pharmaceutical dosage form having a highly hydrophilic fill material and a shell encapsulating the fill material. The shell has at least one plasticizing agent that may have a solubility in the fill material of less than about 10% w/w. US 2016279056 A1 discloses oral pharmaceutical compositions for chewing, sucking, or buccal dissolution comprising soft gel capsules and liquid fills.

So far there is still a need to develop new formulations for an oral pharmaceutical composition containing nalbuphine, to improve the shortcomings of the existing formulations.

### SUMMARY OF THE INVENTION

Accordingly, the present invention provides a novel oral soft capsule that encapsulates a nalbuphine formulation so as to solve the disadvantages of conventional oral pharmaceutical composition containing nalbuphine.

In one aspect, the present invention provides an oral soft capsule that encapsulates a nalbuphine formulation, each soft capsule comprising:
(1) a nalbuphine formulation comprising:
   - 1 mg ~ 1000 mg of nalbuphine; and
   - 100 mg ~ 10 g of an excipient, selected from the group consisting of:
      polysorbate 20, polyethylene glycol 400 (PEG 400) and combinations thereof; and
(2) a capsule shell consisting essentially of:
   - 10 mg ~ 3 g of gelatin;
   - 5 mg ~ 500 mg of a sorbitol component selected from the group consisting of sorbitol, sorbitan and combination thereof, in a percentage of not less than 4% but less than 18% by weight based on the total weight of the capsule shell; and
   - 10 mg ~ 2 g of glycerin The soft capsule may have a shelf life of more than one year.

In some embodiments of the present invention, the oral soft capsule that encapsulates a nalbuphine formulation, each soft capsule comprising:
(1) a nalbuphine formulation comprising:
   - 10 mg ~ 300 mg of nalbuphine; and
   - 100 mg ~ 2.5 g of an excipient, selected from the group consisting of polysorbate 20, PEG 400 and combinations thereof; and
(2) a capsule shell consisting essentially of:
   - 30 mg ~ 1 g of gelatin;
   - 10 mg ~ 300 mg of a sorbitol component selected from the group consisting of sorbitol, sorbitan and combination thereof, in a percentage of not less than 4% but less than 18% by weight based on the total weight of the capsule shell; and
   - 20 mg ~ 660 mg of glycerol The soft capsule may have a shelf life of more than one year.

In one particular example of the present invention, the oral soft capsule that encapsulates a nalbuphine formulation, each soft capsule comprising:
(1) a nalbuphine formulation comprising:
   - 20 mg ~ 120 mg of nalbuphine; and
   - 100 mg ~ 1 g of an excipient, selected from the group consisting of polysorbate 20, PEG 400 and combinations thereof; and
(2) a capsule shell comprising as main components:
   - 60 mg ~ 100 mg of gelatin;
   - 12 mg ~ 120 mg of a sorbitol component selected from the group consisting of sorbitol, sorbitan and combination thereof, in a percentage of not less than 4% but less than 18% by weight based on the total weight of the capsule shell; and
   - 10 mg ~ 2 g of glycerin.
   The soft capsule may have a shelf life of more than one year.

In some embodiments of the present invention, the soft capsule has a shelf life of more than 3 years.

In one example, the sorbitol component is sorbitol. In another example, the sorbitol component is sorbitan. In one particular embodiment of the present invention, the sorbitol component is a combination of sorbitol and sorbitan.

In one embodiment of the present invention, the sorbitol component is present in a percentage of 6% ~ 18% but less than 18%, preferably 6% ~ 15%, by weight of the capsule shell.

In one particular example of the present invention, the sorbitol component is present in a percentage of about 6% by weight based on the total weight of the capsule shell.

In another particular example of the present invention, the sorbitol component is present in a percentage of about 12% by weight based on the total weight of the capsule shell.

In the other particular example of the present invention, the sorbitol component is present in a percentage of about 15% by weight based on the total weight of the capsule shell.

In one embodiment of the invention, the oral soft capsule that encapsulates a nalbuphine formulation further comprises isorhamnetin. In one example, each soft capsule contains isorhamnetin in an amount of 12.5 mg ~ 50 mg.

According to an embodiment of the present invention, the oral soft capsule that encapsulateing a nalbuphine formulation has a capsule shell each consistings essentially of the following components:
- 60 mg ~ 400 mg of gelatin,
- 12 mg ~ 120 mg of a combination of sorbitol and sorbitan ,
- 40 mg ~ 250 mg of glycerol; and
- 18mg ~ 62 mg of pure water.

According to an embodiment of the present invention, the oral soft capsule that encapsulating a nalbuphine formulation has a capsule shell each consisting of the following components:
- 60 mg ~ 400 mg of gelatin,
- 12 mg ~ 120 mg of a combination of sorbitol and sorbitan,
- 40 mg ~ 250 mg of glycerol;
- 18 mg ~ 62 mg of purified water; and
- a flavour agent and a whitening agent.

In one embodiment of the present invention, the oral soft capsule that encapsulates a nalbuphine formulation may optionally comprise isorhamnetin, in the amount of 7.5 mg ~ 200 mg, preferably 7.5 mg ~ 50 mg, more preferably 12.5 mg ~ 50mg in each soft capsule.

In one embodiment of the present invention, the soft capsule further comprises a buffering agent, an antibacterial and/or preservative agent. In one example, the buffering agent may be sodium citrate and citric acid; the antibacterial and/or preservative agent may be methylparaben or propylparaben or combinations thereof.

In one embodiment of the present invention, the oral soft capsule that encapsulates a nalbuphine formulation is for use in treating pain.

Also provided is a method for preparing the oral soft capsule that encapsulates a nalbuphine formulation as described herein to prolong shelf life, comprising:
(1) the steps of preparing a nalbuphine formulation, including:
   - mixing an excipient, an antibacterial and/or preservative agent, pure water and a buffering agent with stirring until dissolution to a clear solution, wherein the excipient is selected from the group consisting of polysorbate 20, PEG 400 and combinations thereof; and
   - sieving nalbuphine and adding the sieved nalbuphine to the clean solution to obtain the nalbuphine formulation;
(2) the steps of preparing a capsule shell including:
   - mixing pure water, glycerin, sorbitol and gelatin with stirring and heating to 50 ~ 70 °C until homogeneous such that the sorbitol is present in a percentage not less than 4% but less than 18% by weight based on the total weight of the capsule shell; and
   - continuously stirring and vacuum defoaming until the pressure of 50 ~ 60 cmHg to complete the preparation of the capsule shell;
(3) the steps of granulation including:
   - granulating empty capsules, drying, filling with the nalbuphine formulation to obtain the soft capsules, and sub-packaging the oral soft capsules as obtained;
   whereby the soft capsules have a shelf life of more than one year, particularly 3 years or more.

It should be understood that both the foregoing abstract description and the following detailed description are illustrative and explanatory only, rather than limiting the scope of the present invention. The invention shall be defined by the appended set of claims, solely.

### BRIEF DESCRIPTION OF THE DRAWINGS

The foregoing summary, together with the following detailed description of the invention, will be better understood when read in conjunction with the accompanying drawings. In order to illustrate the present invention, presently preferred embodiments are shown in the drawings.

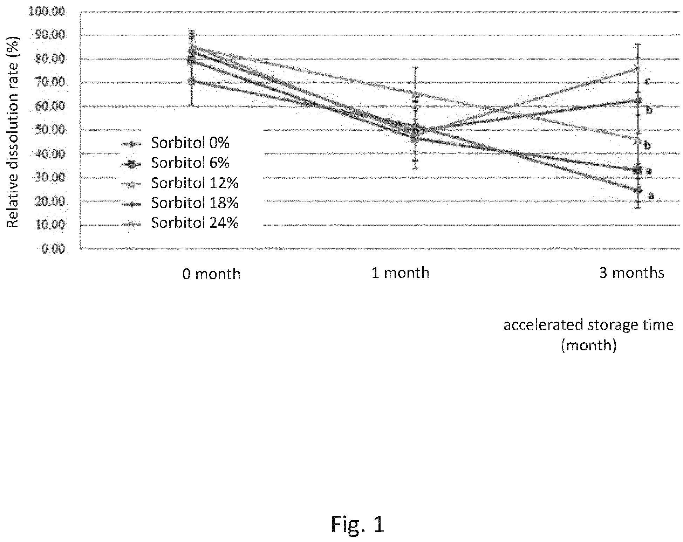

Fig. 1 shows the comparison in the relative dissolution rate (10-minute relative solubility (%)) over the storage times of the oral soft capsules encapsulating a nalbuphine formulation, having various formulae of the capsule shells (containing sorbitol 6 %, sorbitol 12 %, sorbitol 18 % and sorbitol 24 %) in aluminum foil sheet packages; wherein the English characters (a, b, c) marked on the figure represent the significant difference (P<0.05) between the test group and the control group (sorbitol 0%).

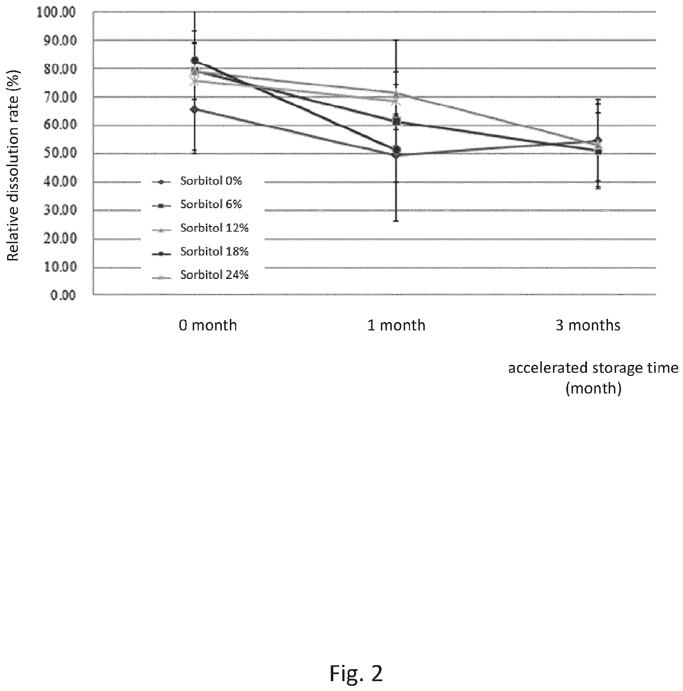

Fig. 2 shows the comparison in the relative dissolution rate (10-minute relative solubility (%)) over the storage time of the oral soft capsules encapsulating a nalbuphine formulation, having various formulae of the capsule shells (containing sorbitol 6 %, sorbitol 12 %, sorbitol 18 % and sorbitol 24 %) in bottled packages.

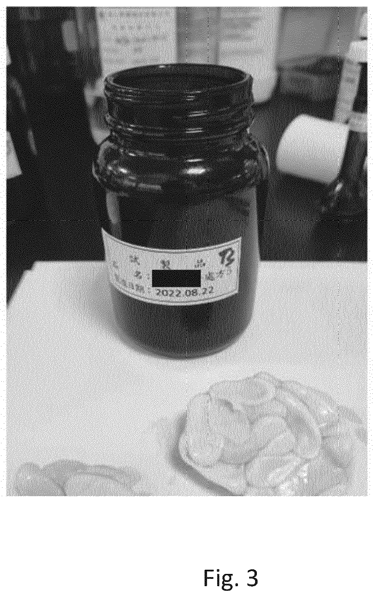

Fig. 3 shows the appearance of the oral soft capsules encapsulating a nalbuphine formulation in bottled packages after a three-month accelerated storage test, wherein the appearance of the capsule shell containing 24% sorbitol was sticking and cracking, and thus the shelf life was shortened.

### DETAILED DESCRIPTION OF THE INVENTION

Unless defined otherwise, all technical and scientific terms used herein have the same meaning as commonly understood by one skilled in the art to which this invention belongs.

The present invention is further illustrated by the following examples, which are intended to be provided as illustrations and not as limitations of the invention.

As used herein, the "a" article refers to one or more than one (i.e., at least one) of the grammatical object of the article. By way of example, "a component" means one component or more than one component.

The present invention provides a solution to the problems of conventional oral pharmaceutical composition containing nalbuphine as an active ingredient. Nalbuphine has low bioavailability, by using specific combination of excipients to improve the bioavailability of nalbuphine in an oral preparation, while such excipients cause the problem of hardening the capsule crust and by adding sorbitol to improve the hardening problem in turn leads to the sticking and cracking problems of the capsule crust. To improve poor shelf life due to the sticking and cracking problems, the present invention modifies the formulae of the capsule shell to prepare an oral soft capsule having both improved bioavailability and prolonged shelf life. The purposes of the present invention include to improve the oral absorption of nalbuphine which is of low bioavailability, and to improve the physical properties of the shell of a soft capsule so as to prolong the shelf life.

Accordingly, the present invention provides an oral soft capsule that encapsulates a nalbuphine formulation, each soft capsule comprising:
(1) a nalbuphine formulation comprising:
   - nalbuphine, in an amount of 1 mg ~ 1000 mg, preferably 10 mg ~ 300 mg, more preferably 20 mg ~ 120 mg; and
   - an excipient, selected from the group consisting of: polysorbate 20, PEG 400 and combinations thereof, in an amount of 100 mg ~10 g, preferably 100 mg ~ 2.5g, more preferably 120mg ~ 1g;
(2) a capsule shell comprising as main ingredients:
   - gelatin, in an amount of 10 mg ~ 3 g, preferably 30 mg ~ 1 g, more preferably 60 mg ~400 mg;
   - a sorbitol component selected from the group consisting of sorbitol, sorbitan and combination thereof, in an amount of 5 mg ~ 500 mg, preferably 10 mg ~ 300 mg, more preferably 12 mg ~ 120 mg; and;
   - glycerin, in an amount of 10 mg ~ 2 g, preferably 20 mg ~ 660 mg, more preferably 40 mg ~ 250 mg;
   the sorbitol component is present in a percentage not less than 4% but less than 18% by weight based on the total weight of the capsule shell. The soft capsule may have a shelf life of more than one year.

In the present invention, nalbuphine is used as an active ingredient, which is
a kappa-opioid receptor agonist and a partial mu-opioid receptor antagonist. Because of this unique mixed agonist-antagonist opioid receptor activity of nalbuphine, it provides analgesia with less nausea, pruritus, and respiratory depression when compared to morphine. In addition, nalbuphine has very low addiction, no cardiovascular side effects, so that its clinical use is very wide. Nalbuphine has a structural formula below:

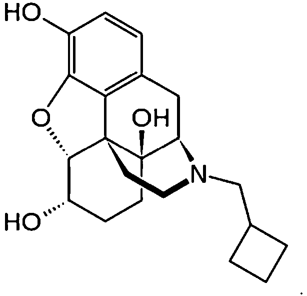

In some embodiments of the invention, an excipient as used herein is polysorbate 20 (also known as Tween 20), PEG 400 or combinations thereof, which provides the effects in stabilizing emulsified and suspended solutions and is often used as a lubricant, and a solubilizer and a wetting agent in pharmaceutical preparations, so as to improve oral bioavailability of nalbuphine.

According to the present invention, the oral soft capsule that encapsulates a nalbuphine formulation may be supplemented with isorhamnetin to increase the bioavailability of nalbuphine, in an amount of 7.5 mg ~ 200 mg, preferably 7.5 mg ~ 50 mg, more preferably 12.5 mg ~ 50 mg in each soft capsule. Isorhamnetin, also known as 3,5,7,4'-tetrahydroxy-3'-methoxyflavone (3,5,7,4' -Tetrahydroxy-3'-methoxyflavone), is a quercetin- derived O - methylated flavonol having the structural formula below:

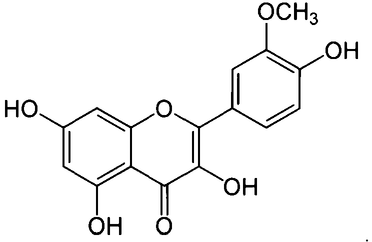

According to Taiwan Patent No. I397413, the human clinical experiments demonstrate that nalbuphine combined with excipients including polysorbate 20 and PEG 400 show the best bioavailability, and that of the composition having natural isorhamnetin is superior to others. The comparison shows that 66 mg of nalbuphine combined with 240 mg of polysorbate 20, 240mg of PEG 400 and 50 mg of isorhamnetin is the optimal composition, whereby Cₘₐₓ can be increased by about 2.5 times, and AUC can be increased by about 2 times.

According to the present invention, the nalbuphine formulation may further include a buffering agent, an antibacterial and/or preservative agent, a thickener, a dispersant and the like. The buffering agent can be sodium citrate and citric acid; the antibacterial and/or preservative agent can be methylparaben and/or propylparaben. PEG400 can act as a dispersant and a thickening agent.

In the nalbuphine formulation of the soft capsule, polysorbate 20, PEG400 or combinations thereof are used as excipients; however, the quality and appearance of capsule shell would be affected during the shelf storage. In the present invention, it is unexpectedly found that the capsule shell comprises/consists essentially of/consists of gelatin, sorbitol, a sorbitol component, glycerol and others including a flavour (such as vanillin) and a whitening agent (such as titanium dioxide) so as to improve the physical properties.

"Gelatin" as used in the present invention has various characteristics such as gelatinity, water fixation, cohesiveness and solubility, and can be used as a gelling agent for a capsule shell.

"Glycerin" as used in the present invention may be added to a capsule shell to avoid drying due to water evaporation.

"A sorbitol component" as used in the present invention includes sorbitol or sorbitan. The term "sorbitan" as used herein refer to a mixture of isomers produced by dehydration of sorbitol which is an intermediate in the conversion of sorbitol to isosorbide.

According to the present invention, in order to solve the problem of long-term low bioavailability of a soft capsule that encapsulates a nalbuphine formulation, polysorbate 20, polyethylene glycol 400 (PEG 400) or combinations thereof is/are used to enhance bioavailability of nalbuphine, which however makes the quality of the soft capsule shell poor. The present invention features by using an appropriate amount of a sorbitol component in the capsule shell to maintain the dissolution rate of nalbuphine , to enhance the surface gloss of the capsule shell, to prevent leakage and to provide consistent the quality of nalbuphine, and to further maintain an appropriate moisture balance within the soft capsule, and to solve the sticking and cracking problems of the capsule shell during shelf storage caused by the using of polysorbate 20, polyethylene glycol 400 (PEG 400) or combinations thereof in the nalbuphine formulation.

According to the present invention, to provide the best quality of capsules, without the sticking and cracking problems of the capsule shell and to provide a longer shelf life, a sorbitol component is used in a certain amount, not less than 4% but less than 18% by weight based on the total weight of the capsule shell, to provide the best quality of capsules, without the sticking and cracking problems of the capsule shell and provide a longer shelf life. In one embodiment, the sorbitol component is present in a percentage of 6% ~ 18%, but less than 18%, by weight based on the total weight of the capsule shell. In one preferred embodiment, the sorbitol component is present in a percentage of 6% ~ 15% by weight based on the total weight of the capsule shell. For example, the sorbitol component may be present in a percentage of about 6%, about 12% or about 15% by weight based on the total weight of the capsule shell.

Also provided is a method for preparing the oral soft capsule that encapsulates a nalbuphine formulation according to the invention to prolong the shelf life, comprising three steps of preparing a nalbuphine formulation, preparing a capsule shell and granulation. The method of the present invention comprises the following steps:
(1) the steps of preparing a nalbuphine formulation including:
   - mixing an excipient, an antibacterial and/or preservative agent, pure water and a buffering agent with stirring until dissolution to a clear solution, wherein the excipient is selected from the group consisting of polysorbate 20, PEG 400 and combinations thereof;
   - sieving nalbuphine and adding the sieved nalbuphine to the clean solution to obtain the nalbuphine formulation;
(2) the steps of preparing a capsule shell including:
   - mixing pure water, glycerin, sorbitol and gelatin with stirring and heating to 50 ~ 70 °C until homogeneous such that sorbitol is present in a percentage not less than 4% but less than 18% by weight based on the total weight of the capsule shell;
   - continuously stirring and vacuum defoaming until the pressure of 50 ~ 60 cmHg to complete the preparation of the capsule shell;
(3) the steps of granulation including:
   - granulating empty capsules, drying, filling with the nalbuphine formulation to obtain the soft capsules, and sub-packaging the soft capsules as obtained;
   whereby the soft capsule has a shelf life of more than one year.

The method for preparing the oral soft capsule that encapsulates a nalbuphine formulation according to the invention to prolong shelf life, may comprise the following steps:
(1) the steps of preparing a nalbuphine formulation including:

| | |
|---|---|
| Step 1: | heating the mixing tank to 60 ~ 70°C first, and adding the first excipient polysorbate 20 into the mixing tank with stirring to dissolve it; |
| Step 2: | stopping heating and adding an antibacterial and/or preservative agent into the mixing tank with stirring to dissolve it; |
| Step 3: | then adding pure water, the second excipient polyethylene glycol 400 and a buffering agent into the mixing tank with stirring using a stirring bar until dissolution to a clear solution; and |
| Step 4: | Sieving nalbuphine through the mesh using a vibrating filter sieve machine and then adding the sieved nalbuphine to the mixing tank with stirring to make it evenly mixed; |
| | and |

(2) the steps of preparing a capsule shell including:

| | |
|---|---|
| Step 1: | adding pure water, glycerin, sorbitol (such as 6%, 12%, 18%, 24%) and gelatin into a preparation tank with stirring and continuous heating to 50 ~ 70°C, the whole mixing time being about 40 ~ 60 minutes; and |
| Step 2: | vacuuming: continuous stirring and vacuum defoaming until the pressure reaching 50 ~ 60 cmHg, the defoaming time being about 10 ~ 30 minutes, to complete the capsule shell mixing work; and |

(3) the steps of granulation including:
granulating empty capsules, drying, filling with the nalbuphine formulation to obtain the soft capsule, and sub-packaging the soft capsule as obtained.

An accelerated test may be utilized to evaluate the shelf life of the product. In the Evaluation Guidelines for Effective Date of Packaged Food on the Market announced on April 24, 2013, it is mentioned that for products with a long expiry date, an accelerated shelf life studies can be used to estimate the expiry date. In general, an accelerated test by using an increased temperate for storage to accelerate deterioration of products and the expiration date of the product can be estimated under the storage conditions as set. Normally, a 3-month accelerated test indicates a 12-month actual period of time. The products prepared according to the present invention have been evaluated by the accelerated studies for 3 months where quite good quality is maintained. It has been proved that the shelf life can reach more than 1 year, and the results of the actual product shelf life studies show that the shelf life can be extended for more than 3 years.

The present invention further illustrates the technical characteristics of the present invention with the following examples, but it is not intended to limit the scope of the present invention.

### Example

### Example 1. Preparation of the soft capsule of the present invention

### 1.1 Preparation steps for a nalbuphine formulation:

| | |
|---|---|
| Step 1: | heating the mixing tank to 60-70°C first, and adding the first excipient polysorbate 20 into the mixing tank with stirring to dissolve it. |
| Step 2: | stopping heating and adding an antibacterial and/or preservative agent into the mixing tank with stirring to dissolve it. |
| Step 3: | then adding pure water, the second excipient polyethylene glycol 400 and a buffering agent into the mixing tank with stirring using a stirring bar until dissolution to a clear solution. |
| Step 4: | Sieving nalbuphine through the mesh using a vibrating filter sieve machine and then adding the sieved drug to the mixing tank with stirring to make it evenly mixed. |

### 1.2 Preparation steps for a capsule shell with sorbitol in various percentages

| | |
|---|---|
| Step 1: | adding pure water, glycerin, sorbitol (such as 6%, 12%, 18%, 24%) and gelatin into a preparation tank with stirring and continuous heating to 50 ~ 70°C, the whole mixing time being about 40 ~ 60 minutes. |
| Step 2: | vacuuming: continuous stirring and vacuum defoaming until the pressure reaching 50 ~ 60 cmHg, the defoaming time being about 10 ~ 30 minutes, to complete the capsule shell mixing work. |

### 1.3 the steps of granulation:

granulating empty capsules, drying, filling with the nalbuphine formulation to obtain the soft capsule, and sub-packaging the soft capsule as obtained.

### Example 2. Dissolution test of the oral soft capsules encapsulating a nalbuphine formulation according to the present invention

2.1 The comparison index standard of dissolution was as follows:
Solvent: solution pH1.2
Dissolution time: 10 minutes, 20 minutes, 30 minutes.
Number of samples: 6 soft capsules.

2.2 Samples were tested and grouped as follows:
There were five (5) test groups in total, each test group for two packages (aluminum foil sheet and bottle). The 5 test groups were provided for the different formulae of the capsule shell containing sorbitol 0%, 6%, 12%, 18% and 24%, respectively. Three test time points include the first beginning, called as the month zero (T0) in the accelerated study, the first month (T1) in the accelerated study and the third month (T3) in the accelerated study. However, there was no need to split into two packages for the time point of the zero month (T0), where only one package condition was tested.

### 2.3 Standard Operating Procedures for Analytical Methods

### 2.3.1 Dissolution conditions

Solvent: Solution pH1.2: 900mL
Temperature: 37°C + 0.5°C.
Speed: 50 rpm
Duration: 10 minutes, 20 minutes, 30 minutes
Number of samples: 6 capsules

### 2.3.2 Operation mode:

The dissolution test was performed and the timer was counted. When the test time limit was reached, 2 ml of the test sample in the middle of the rotation axis and the test tank wall and one-half of the solvent at the predetermined sampling time was drawn and then 2 ml of solvent was replenished. The test sample was filtered and the filtrate was taken directly for testing.
HPLC conditions: detection of nalbuphine HCl.
Chromatography column: GL Sciences Inertsil ODS-3 (5µm) 4.6*250mm
Light wavelength of detection: 280 nm
Flow rate: 1.3 mL/min
Injection volume: 100 µL
Mobile phase:
Buffer 1.08 g PICB8 + 14.35g CH3COONa (23.8g CH 3 COONa • 3H 2 O) + l mL TEA+25mL G-HOAc -> in H 2 O 1000mL
Mobile Phase A: Buffer/ACN = 95/5 Mobile Phase B: Buffer/ACN = 50/50

| time | mobile phase A | mobile phase B |
|---|---|---|
| 0.01 | 85% | 15% |
| 15.0 | 65% | 35% |
| 30.0 | 10% | 90% |
| 41.0 | 10% | 90% |
| 42.0 | 85% | 15% |
| 55.0 | 85% | 15% |

Diluent: Mobile Phase A

### 2.3.3 Test medium configuration:

pH1.2 solvent: 2 g NaCl was dissolved in 500 mL of water, 7.0 mL of concentrated hydrochloric acid (38%) was added and water was added to 1000 mL.

### 2.3.4 Standard solution configuration:

33.0 mg of nalbuphine HCl was accurately weighed and added to a 50 mL brown quantitative bottle and a diluent was added to a graduation for quantification. 1mL of the above solution was taken out, put it in a 10 mL quantitative bottle, and a diluent was added to a graduation for quantification. The mixture was mixed well and filtered with a 0.45 µm filter membrane. This solution is the standard solution.

### 2.3.5 System suitability:

The standard solution was injected into the HPLC and the chromatographic peaks were recorded. The injection was repeated with 6 needles RSD < 2.0%.

2.3.6 Analysis and comparison of the spectra obtained from the standard substance and the test solution were performed.

2.3.7 The above steps were repeated for different batches of samples for data analysis.

### 2.4 Results

The results of the test samples in the aluminum foil sheet packages and bottled packages are shown in Figs. 1 and 2. Fig. 1 shows the results of the aluminum foil sheet packages for groups of various sorbitol formulae (sorbitol 0%, 6%, 12%, 18%, 24%) where the 10-minute relative solubility (%) varies with the accelerated storage time (T0 (0 month), T1(1 month) and T3 (3 months)) that the group of 12% sorbitol shows the best 10-minute relative solubility (%); while the 10-minute relative solubility (%) shows an upward trend in the T3 (3 months) accelerated storage time when the sorbitol amount in the capsule shell increases (the English letters (a, b, c) marked on the figure represent a significant difference (P<0.05) compared with the control group (sorbitol 0 % group).

Fig. 2 shows the results of the tests for bottled samples. In the groups of the formulae containing sorbitol 18% and 24%, the dissolution rate could not be measured because the capsules were sticky and cracked. The results show that for groups of various sorbitol formulae (sorbitol 0%, sorbitol 6%, sorbitol 12%, sorbitol 18%, sorbitol 24%), the 10-minute relative solubility (%) varies with the accelerated storage time (T0 (0 month), T1(1 month) and T3 (3 months) that the group of 12% sorbitol shows the best 10-minute relative solubility (%); while the 10-minute relative solubility (%) shows an downward trend in the T3 (3 months) accelerated storage time for groups of sorbitol 0%, 6% and 12%, and the dissolution rate could not be measured for groups of sorbitol 18% and sorbitol 24% because the capsules were cracked and sticky to clumps. It can be seen that for the 10-minute relative solubility (%), a higher amount of sorbitol may not necessarily bring the better results.

Further, the impact of the sorbitol amount on the physical properties of the capsule appearance was observed, for example, the capsule being sticky, cracked, deformed or leaky etc.
The observation results are shown in Table 1.

As shown in Table 1, the soft capsules containing excipients absorbed moisture and the capsule shells became hardened when no sorbitol (0%) was added. Therefore, sorbitol was added in order to improve the problem of hardening the capsule shell. However, when the sorbitol amount was up to 24%, sticking and cracking problems occurred on the capsule shell after storage in the bottles in the accelerated storage studies (1 or 3 months). As shown in Fig. 3, when the sorbitol amount of the capsule shell was up to 24%, the appearance of the capsule shell surface shows sticking and cracking in the accelerated studies for 3 months that the shelf life was shortened, affecting the shelf life. Therefore, it is necessary to choose an appropriate amount of sorbitol (4-18 %).
Table 1 shows the effects of sorbitol amounts on the physical properties of capsule appearance in the accelerated storage studies (0, 1 or 3 months).

| Capsule shell formulat ion Sorbitol % ( w/w) | Package/Cont ainer | 0 month (T0) capsule appearance | 1 month (T1) capsule appearance | 3 months (T3) capsule appearance |
|---|---|---|---|---|
| 0% | bottled packages | no sticking/cracking/defo rmation problems | no stickiness, slightly hardened capsule shell | no stickiness, capsule shell being slightly hardened, some appearing wrinkled. |
| 0% | aluminum foil sheet packages | no sticking/cracking/defo rmation problems | capsule shell being slightly hardened | capsule shell being slightly hardened, some appearing wrinkled. |
| 6% | bottled packages | no sticking/cracking/defo rmation problems | no sticking/cracking/defo rmation problems | capsules being slightly sticky, no deformation or cracking problems |
| 6% | aluminum foil sheet package | no sticking/cracking/defo rmation problems | no sticking/cracking/defo rmation problems | no sticking/cracking/defo rmation problems |
| 12% | bottled packages | no sticking/cracking/defo rmation problems | no sticking/cracking/defo rmation problems | capsules being slightly sticky, no deformation or cracking problems |
| 12% | aluminum foil sheet package | no sticking/cracking/defo rmation problems | no sticking/cracking/defo rmation problems | no sticking/cracking/defo rmation problems |
| 18% | bottled packages | no sticking/cracking/defo rmation problems | capsule shell being slightly hardened and deformed (slightly flat) | capsules being sticky to each other and cracked easily when separated with tweezers |
| 18% | aluminum foil sheet package | no sticking/cracking/defo rmation problems | no sticking/cracking/defo rmation problems | capsule shell being slightly deformed (slightly flat) |
| 24% | bottled packages | no sticking/cracking/defo rmation problems | capsule shell being slightly hardened and deformed (slightly flat) | capsules being sticky to each other and already broken when separated with tweezers |
| 24% | aluminum foil sheet package | no sticking/cracking/defo rmation problems | capsule shell being slightly deformed (slightly flat) | capsule shell being obviously deformed (flatter) |

To sum up, proper amounts of sorbitol (4% ~ 18 %, such as 6%, 12% or 15%) are added to the capsule shell according to the present invention which can increase the dissolution rate and improve the problems of capsule shells such as sticking and cracking so as to prolong the shelf life.

### Example 3. The long-term shelf life test of the oral soft capsules encapsulating a nalbuphine formulation according to the present invention

To evaluate the shelf life of the oral soft capsules encapsulating a nalbuphine formulation according to the present invention, the samples of the aluminum foil sheet packages prepared according to the process of the present invention were tested, each capsule containing 20 mg ~ 120 mg of nalbuphine, 120 mg ~ 1 g of polysorbate 20 and PEG 400 as excipients, and 60 mg ~ 400 mg of gelatin, 12 mg ~ 120 mg of a combination of sorbitol and sorbitan, and 40 mg ~ 250 mg of glycerin, as the main components of the capsule shell, wherein the sorbitol component (which is a combination of sorbitol and sorbitan) was present in a percentage be not less than 4% but less than 18% by weight of the total weight of the capsule shell. The test time points were 1, 3, 6, 9, 12, 18, 24 and 36 months. The test conditions were 25 ± 2 °C / 60% RH ± 5%. These test results are shown in Table 2.

**Table 2: Shelf life test of the soft capsule of the present invention**

| Test conditions | 1 month | 3 months | 6 months | 9 months | 12 months |
|---|---|---|---|---|---|
| Capsule appearance | no sticking/cracking /deformation problems | no sticking/cracking /deformation problems | no sticking/cracking /deformation problems | no sticking/cracking /deformation problems | no sticking/cracking /deformation problems |
| Dissolution rate greater than 80.0% tested in 30 minutes | minimum: 102.0% | minimum: 95.8% | minimum: 101.7% | minimum: 97.8% | minimum: 94.3% |
| Results | Qualified | Qualified | qualified | qualified | qualified |

| Test Conditions | 18 months | 24 months | 36 months |
|---|---|---|---|
| capsule appearance | no sticking/cracking/ deformation problems | no sticking/cracking/ deformation problems | no sticking/cracking/ deformation problems |
| Dissolution rate greater than 80.0% tested in 30 minutes | minimum: 98.09% | minimum: 96.77% | minimum: 99.83% |
| Results | Qualified | qualified | qualified |

As shown in Table 2, the soft capsules encapsulating a nalbuphine formulation according to the present invention comprising 4 ~ 18 % of the sorbitol component maintained consistent dissolution rate or appearance for 1 month to 36 months with qualified results, showing that the shelf life lasted up to 3 years or more.

Although the present invention provides the above preferred embodiments, it is not intended to limit the present invention. The scope of protection of the present invention should be defined by the appended claims.

## Claims

1. An oral soft capsule that encapsulates a nalbuphine formulation, each soft capsule comprising:
(1) a nalbuphine formulation comprising:
- 1 mg ~ 1000 mg of nalbuphine; and
- 100 mg ~ 10 g of an excipient, selected from the group consisting of:
polysorbate 20, polyethylene glycol 400 (PEG 400) and combinations thereof; and
(2) a capsule shell consisting essentially of:
- 10 mg ~ 3 g of gelatin;
- 5 mg ~ 500 mg of a sorbitol component selected from the group consisting of sorbitol, sorbitan and combination thereof, in a percentage of not less than 4% but less than 18% by weight based on the total weight of the capsule shell; and
- 10 mg ~ 2 g of glycerin.

2. The oral soft capsule that encapsulates a nalbuphine formulation according to claim 1, each soft capsule comprising:
(1) a nalbuphine formulation comprising:
- 10 mg ~ 300 mg of nalbuphine; and
- 100 mg ~ 2.5 g of an excipient, selected from the group consisting of polysorbate 20, PEG 400 and combinations thereof; and
(2) a capsule shell consisting essentially of:
- 30 mg ~ 1 g of gelatin;
- 10 mg ~ 300 mg of the sorbitol component; and
- 20 mg ~ 660 mg of glycerin.

3. The oral soft capsule that encapsulates a nalbuphine formulation according to claim 2, each soft capsule comprising:
(1) a nalbuphine formulation comprising:
- 20 mg ~ 120 mg of nalbuphine; and
- 120 mg ~ 1 g of an excipient, selected from the group consisting of polysorbate 20, PEG 400 and combinations thereof; and
(2) a capsule shell consisting essentially of:
- 60 mg ~ 400 mg of gelatin;
- 12 mg ~ 120 mg of the sorbitol component; and
- 40 mg ~ 250 mg of glycerin.

4. The oral soft capsule that encapsulates a nalbuphine formulation according to any one of claims 1-3, wherein the sorbitol component is a combination of sorbitol and sorbitan.

5. The oral soft capsule that encapsulates a nalbuphine formulation of any one of claims 1-3, wherein the sorbitol component is present in a percentage of 6% - 18%, but less than 18%, by weight based on the total weight of the capsule shell.

6. The oral soft capsule that encapsulates a nalbuphine formulation according to claim 5, wherein the sorbitol component is present in a percentage of 6% - 15% by weight based on the total weight of the capsule shell.

7. The oral soft capsule that encapsulates a nalbuphine formulation according to claim 6, wherein the sorbitol component is present in a percentage of about 6% by weight based on the total weight of the capsule shell; or
wherein the sorbitol component is present in a percentage of about 12% by weight based on the total weight of the capsule shell; or
wherein the sorbitol component is present in a percentage of about 15% by weight based on the total weight of the capsule shell.

8. The oral soft capsule that encapsulates a nalbuphine formulation according to any one of claims 1-7, which further comprises isorhamnetin.

9. The oral soft capsule that encapsulates a nalbuphine formulation according to claim 8, wherein each soft capsules comprises 12.5 mg ~ 50 mg of isorhamnetin.

10. The oral soft capsule that encapsulates a nalbuphine formulation according to any one of claims 1-9, which further comprises a buffering agent, and an antibacterial and/or a preservative agent.

11. The oral soft capsule that encapsulates a nalbuphine formulation according to any one of claims 1-10, which is **characterized in that** the capsule shell of each soft capsule consists essentially of:
- 60 mg ~ 400 mg of gelatin;
- 12 mg ~ 120 mg of the sorbitol component, being a combination of sorbitol and sorbitan; and
- 40 mg ~ 250 mg of glycerol; and
- 18 mg ~ 62 mg of pure water.

12. The oral soft capsule that encapsulates a nalbuphine formulation according to any one of claims 1-10, which is **characterized in that** the capsule shell of each soft capsule consists of:
- 60 mg ~ 400 mg of gelatin,
- 12 mg ~ 120 mg of the sorbitol component, being a combination of sorbitol and sorbitan,;
- 40 mg ~ 250 mg of glycerin;
- 18 mg ~ 62 mg of pure water; and
- a flavour and a whitening agent.

13. The oral soft capsule that encapsulates a nalbuphine formulation according to any one of claims 1-12, which is for use in treating pain.

## Patentansprüche

1. Orale Weichkapsel, welche eine Nalbuphinformulierung enthält, worin jede Weichkapsel umfasst:
(1) eine Nalbuphinformulierung, die umfasst:
- 1 mg - 1000 mg Nalbuphin; und
- 100 mg - 10 g eines Hilfsstoffs, ausgewählt aus der Gruppe bestehend aus: Polysorbat 20, Polyethylenglykol 400 (PEG 400) und Kombinationen davon; und
(2) eine Kapselhülle, die im Wesentlichen besteht aus:
- 10 mg - 3 g Gelatine;
- 5 mg - 500 mg einer Sorbitolkomponente, ausgewählt aus der Gruppe bestehend aus Sorbitol, Sorbitan und Kombinationen davon, in einem Anteil von nicht weniger als 4 Gew.-%, jedoch weniger als 18 Gew.-%, bezogen auf das Gesamtgewicht der Kapselhülle; und
- 10 mg - 2 g Glycerin.

2. Orale Weichkapsel, welche eine Nalbuphinformulierung gemäß Anspruch 1 enthält, worin jede Weichkapsel umfasst:
(1) eine Nalbuphinformulierung, die umfasst:
- 10 mg - 300 mg Nalbuphin; und
- 100 mg - 2,5 g eines Hilfsstoffs, ausgewählt aus der Gruppe bestehend aus Polysorbat 20, PEG 400 und Kombinationen davon; und
(2) eine Kapselhülle, die im Wesentlichen besteht aus:
- 30 mg - 1 g Gelatine;
- 10 mg - 300 mg der Sorbitolkomponente; und
- 20 mg - 660 mg Glycerin.

3. Orale Weichkapsel, welche eine Nalbuphinformulierung gemäß Anspruch 2 enthält, worin jede Weichkapsel umfasst:
(1) eine Nalbuphinformulierung, die umfasst:
- 20 mg - 120 mg Nalbuphin; und
- 120 mg - 1 g eines Hilfsstoffs, ausgewählt aus der Gruppe bestehend aus Polysorbat 20, PEG 400 und Kombinationen davon; und
(2) eine Kapselhülle, die im Wesentlichen besteht aus:
- 60 mg - 400 mg Gelatine;
- 12 mg - 120 mg der Sorbitolkomponente; und
- 40 mg - 250 mg Glycerin.

4. Orale Weichkapsel, welche eine Nalbuphinformulierung gemäß einem der Ansprüche 1-3 enthält, worin die Sorbitolkomponente eine Kombination aus Sorbitol und Sorbitan ist.

5. Orale Weichkapsel, welche eine Nalbuphinformulierung gemäß einem der Ansprüche 1-3 enthält, worin die Sorbitolkomponente in einem Anteil von 6 Gew.-% - 18 Gew.-%, jedoch weniger als 18 Gew.-%, bezogen auf das Gesamtgewicht der Kapselhülle, vorliegt.

6. Orale Weichkapsel, welche eine Nalbuphinformulierung gemäß Anspruch 5 enthält, worin die Sorbitolkomponente in einem Gewichtsanteil von 6 Gew.-% - 15 Gew.-%, bezogen auf das Gesamtgewicht der Kapselhülle, vorliegt.

7. Orale Weichkapsel, welche eine Nalbuphinformulierung gemäß Anspruch 6 enthält, worin die Sorbitolkomponente in einem Anteil von etwa 6 Gew.-%, bezogen auf das Gesamtgewicht der Kapselhülle, vorliegt; oder
worin die Sorbitolkomponente in einem Anteil von etwa 12 Gew.-%, bezogen auf das Gesamtgewicht der Kapselhülle, vorliegt; oder
worin die Sorbitolkomponente in einem Anteil von etwa 15 Gew.-%, bezogen auf das Gesamtgewicht der Kapselhülle, vorliegt.

8. Orale Weichkapsel, welche eine Nalbuphinformulierung gemäß einem der Ansprüche 1-7 enthält, worin sie ferner Isorhamnetin enthält.

9. Orale Weichkapsel, welche eine Nalbuphinformulierung gemäß Anspruch 8 enthält, worin jede Weichkapsel 12,5 - 50 mg Isorhamnetin enthält.

10. Orale Weichkapsel, welche eine Nalbuphinformulierung gemäß einem der Ansprüche 1-9 enthält, die ferner ein Puffermittel sowie ein antibakterielles Mittel und/oder ein Konservierungsmittel enthält.

11. Orale Weichkapsel, welche eine Nalbuphinformulierung gemäß einem der Ansprüche 1-10 enthält, **dadurch gekennzeichnet, dass** die Kapselhülle jeder Weichkapsel im Wesentlichen besteht aus:
- 60 mg - 400 mg Gelatine;
- 12 mg - 120 mg der Sorbitolkomponente, bei der es sich um eine Kombination aus Sorbitol und Sorbitan handelt; und
- 40 mg - 250 mg Glycerin; und
- 18 mg - 62 mg reines Wasser.

12. Orale Weichkapsel, welche eine Nalbuphinformulierung gemäß einem der Ansprüche 1-10 enthält, **dadurch gekennzeichnet, dass** die Kapselhülle jeder Weichkapsel besteht aus:
- 60 mg - 400 mg Gelatine,
- 12 mg - 120 mg der Sorbitolkomponente, die eine Kombination aus Sorbitol und Sorbitan ist;
- 40 mg - 250 mg Glycerin;
- 18 mg - 62 mg reines Wasser; und
- einem Aromastoff und einem Weißmacher.

13. Orale Weichkapsel, welche eine Nalbuphinformulierung gemäß einem der Ansprüche 1-12 enthält und zur Behandlung von Schmerzen bestimmt ist.

## Revendications

1. Capsule molle à usage oral renfermant une formulation de nalbuphine, chaque capsule molle comprenant:
(1) une formulation de nalbuphine comprenant:
- 1 mg - 1000 mg de nalbuphine; et
- 100 mg - 10 g d'un excipient, choisi parmi le groupe constitué de:
le polysorbate 20, le polyéthylène glycol 400 (PEG 400) et leurs combinaisons; et
(2) une enveloppe de capsule constituée essentiellement de:
- 10 mg - 3 g de gélatine;
- 5 mg - 500 mg d'un composant à base de sorbitol choisi parmi le groupe constitué du sorbitol, du sorbitane et de leurs combinaisons, en un pourcentage non inférieur à 4% mais inférieur à 18% en poids par rapport au poids total de l'enveloppe de capsule; et
- 10 mg - 2 g de glycérine.

2. Capsule molle à usage oral renfermant une formulation de nalbuphine selon la revendication 1, chaque capsule molle comprenant:
(1) une formulation de nalbuphine comprenant:
- 10 mg - 300 mg de nalbuphine; et
- 100 mg - 2,5 g d'un excipient, choisi parmi le groupe constitué du polysorbate 20, du PEG 400 et de leurs combinaisons; et
(2) une enveloppe de capsule constituée essentiellement de:
- 30 mg - 1 g de gélatine;
- 10 mg - 300 mg du composant à base de sorbitol; et
- 20 mg - 660 mg de glycérine.

3. Capsule molle à usage oral renfermant une formulation de nalbuphine selon la revendication 2, chaque capsule molle comprenant:
(1) une formulation de nalbuphine comprenant:
- 20 mg - 120 mg de nalbuphine; et
- 120 mg - 1 g d'un excipient, choisi dans le groupe constitué par le polysorbate 20, le PEG 400 et leurs combinaisons; et
(2) une enveloppe de capsule constituée essentiellement de:
- 60 mg - 400 mg de gélatine;
- 12 mg - 120 mg du composant à base de sorbitol; et
- 40 mg - 250 mg de glycérine.

4. Capsule molle à usage oral renfermant une formulation de nalbuphine selon l'une quelconque des revendications 1-3, dans laquelle le composant à base de sorbitol est une combinaison de sorbitol et de sorbitane.

5. Capsule molle à usage oral renfermant une formulation de nalbuphine selon l'une quelconque des revendications 1-3, dans laquelle le composant à base de sorbitol est présent à raison de 6% - 18%, mais inférieure à 18%, en poids par rapport au poids total de l'enveloppe de capsule.

6. Capsule molle à usage oral renfermant une formulation de nalbuphine selon la revendication 5, dans laquelle le composant à base de sorbitol est présent à raison de 6% - 15% en poids par rapport au poids total de l'enveloppe de capsule.

7. Capsule molle à usage oral renfermant une formulation de nalbuphine selon la revendication 6, dans laquelle le composant à base de sorbitol est présent à raison d'environ 6% en poids par rapport au poids total de l'enveloppe de capsule; ou
dans laquelle le composant à base de sorbitol est présent à raison d'environ 12% en poids par rapport au poids total de l'enveloppe de capsule; ou
dans laquelle le composant à base de sorbitol est présent à raison d'environ 15% en poids par rapport au poids total de l'enveloppe de capsule.

8. Capsule molle à usage oral renfermant une formulation de nalbuphine selon l'une quelconque des revendications 1-7, qui comprend en outre de l'isorhamnétine.

9. Capsule molle à usage oral renfermant une formulation de nalbuphine selon la revendication 8, dans laquelle chaque capsule molle comprend 12,5 mg - 50 mg d'isorhamnétine.

10. Capsule molle à usage oral renfermant une formulation de nalbuphine selon l'une quelconque des revendications 1-9, qui comprend en outre un agent tampon, ainsi qu'un agent antibactérien et/ou un agent conservateur.

11. Capsule molle à usage oral renfermant une formulation de nalbuphine selon l'une quelconque des revendications 1-10, **caractérisée en ce que** l'enveloppe de capsule de chaque capsule molle est constituée essentiellement de:
- 60 mg - 400 mg de gélatine;
- 12 mg - 120 mg du composant à base de sorbitol, qui est une combinaison de sorbitol et de sorbitane; et
- 40 mg - 250 mg de glycérol; et
- 18 mg - 62 mg d'eau pure.

12. Capsule molle à usage oral renfermant une formulation de nalbuphine selon l'une quelconque des revendications 1-10, **caractérisée en ce que** l'enveloppe de capsule de chaque capsule molle est constituée de:
- 60 mg - 400 mg de gélatine,
- 12 mg - 120 mg du composant à base de sorbitol, qui est une combinaison de sorbitol et de sorbitane;
- 40 mg - 250 mg de glycérine;
- 18 mg - 62 mg d'eau pure; et
- un arôme et un agent blanchissant.

13. Capsule molle à usage oral renfermant une formulation de nalbuphine selon l'une quelconque des revendications 1-12, destinée à être utilisée dans le traitement de la douleur.
